# EUROPEAN PATENT APPLICATION

(11) **EP 3 421 614 A1**
(43) Date of publication of application: **02.01.2019**
(21) Application number: 18178025.5
(22) Date of filing: 29.03.2013
(51) Int. Cl.: C12Q 1/6883

(54) **METHOD AND KIT FOR THE CLASSIFICATION AND PROGNOSIS OF WOUNDS**

(30) Priority: 30.03.2012 WO PCT/IB2012/000906
(62) Divisional of application: 13713867.3
(71) Applicant: Urgo Recherche Innovation et Développement, 21300 Chenove (FR); Centre National de la Recherche Scientifique CNRS, 75794 Paris Cedex 16 (FR); Université Paris Diderot - Paris 7, 75013 Paris (FR); École Normale Supérieure, 75005 Paris (FR)
(72) Inventor: DUGAST DARZACQ, Claire, Berkeley (94707) California (US); DARZACQ, Xavier, Berkeley (94707) California (US); NOIZET, Maïté, 31300 Toulouse (FR); LAGOUTTE, Emilie, 95240 Cormeilles en Parisis (FR); ROEST CROLLIUS, Hugues, 94160 Saint Mande (FR); GRATIGNY, Marlène, 92700 Colombes (FR); BOUSCHBACHER, Marielle, 21220 Chambolle-Musigny (FR)
(74) Representative: Cabinet Plasseraud

(57) **Abstract**

The present invention relates to a method and kit for the classification and prognosis of chronic or non-healing wounds of mammalian, in particular in human. The method uses a molecular marker that enables one to characterize a pathological wound healing such as chronic or non-healing wound.

## Description

### Field of the invention

The present invention relates to a method and kit for the classification and prognosis of wounds of mammalian, in particular in human. The method defines a molecular signature that enables one to characterize a pathological wound healing such as chronic or non-healing wound.

### Background of the invention

The natural wound healing is divided into three sequential phases; each phase is characterized by specific cellular activities: the inflammatory phase, the proliferative phase and the remodeling phase.

The first phase, called the inflammatory phase, begins minutes after injury. The blood vessels rupture induces the clot formation, composed mainly of fibrin and fibronectin. The clot fills partially the lesion and allows the migration of the inflammatory cells within the lesion. The inflammatory cells are recruited to debride the wound. Platelets secrete factors, such as growth factors or cytokines, which induce the recruitment of cells implicated in the wound healing (inflammatory cells such as neutrophils and macrophages, fibroblasts and endothelial cells).

The second phase is called the proliferative phase and corresponds to the development of the granulation tissue. Fibroblasts migrate into the wound area, proliferate and form a new provisional extracellular matrix by secreting extracellular matrix (ECM) proteins. Then endothelial cells migrate to promote the neovascularization or angiogenesis of the lesion. Inside the granulation tissue, fibroblasts activate and differentiate into myofibroblasts, presenting contractile properties thanks to their expression of alpha-smooth muscle actin (similar to that in smooth muscle cells). Myofibroblasts have a key role in wound healing as they provide the contraction of the wound. Finally, keratinocytes migrate from the wound edge, proliferate and differentiate to reconstitute the epidermis.

The last phase of the wound healing process appears after the wound closure. It corresponds to the remodeling of the granulation tissue. The granulation tissue is reorganized, type III collagen is replaced by type I collagen, as normal dermis is principally composed of type I collagen. During this phase, myofibroblasts in excess are eliminated by apoptosis. The last phase of the wound healing is long. One year after injury, the scar is remodeled; it gets less red and thinner.

However, this process is not only complex but fragile; it is susceptible to interruption or failure leading to the formation of chronic or non-healing wounds or formation of abnormal scars. Factors which may contribute to this include diseases (such as diabetes, venous or arterial disease), age, infection or tissue localization.

### Chronic or non-healing wounds

Chronic wounds are a worldwide health problem, in part due to a lack of adequate methods of treatment. In 2010, more than 7 million people worldwide suffered from chronic wounds, and the projected annual increase is at least 10 percent.

Chronic wounds are sometimes non-healing wounds. Common types of chronic wounds include, but are not limited to, venous leg ulcers, diabetic foot ulcers, decubitus ulcers, arterial leg ulcers, those of mixed etiology (venous and arterial) or those with no known etiology. We can also find acute wounds that become chronic as they do not heal correctly.

Non-healing wounds or chronic wounds are a challenge for the patient, the health care professional, and the health care system. They significantly impair the quality of life for millions of people and impart burden on society in terms of lost productivity and health care money.

Wound healing is a dynamic pathway that leads to the restoration of tissue integrity and functions. A chronic wound or non-healing wound develops when the normal reparative process is disturbed. By understanding the biology of wound healing, the physician can optimize the wound healing by choosing the adequate treatment.

In chronic or non-healing wounds, the natural healing process is altered, and thus healing is prolonged, incomplete and sometimes wounds never close. A chronic wound occurs when some factor causes the disruption of the normal inflammatory and proliferative phases. Thus, there is a need for a reliable method for diagnosing a non-healing or chronic wound in order to adapt the best treatment to provide the wound closure and healing. There is also a need for a better care of the wound and for the reduction of the deadline of said care.

In some pathological diseases or specific anatomic localizations, early diagnosis of the potential onset of a wound may help to prevent the development of a wound. In the situation where a wound has already developed, knowledge of the diagnosis or prognosis of a wound may enable patients to receive maximum benefit from therapy. Thus, the treatment of the wound is especially adapted to the wound in its early stage if it presents a risk of not healing correctly.

It would be beneficial to know which patients are susceptible to develop chronic or non-healing wounds and furthermore, if a chronic wound does develop, how likely the patient is to respond to a specific therapy. Thus a critical objective is to identify a diagnostic or prognostic method for chronic or non-healing wounds, so as to provide earlier and improved choices of treatment.

Although some common clinical/pathological factors may assist in pre-judging if a wound may be "healing" or "non-healing", or if an acute wound may become chronic, there is no specific laboratory test(s) to distinguish among wound types. Woundcheck status® commercialized by Systagenix enables one to measure proteases activity but is not specific enough to distinguish between the chronic wounds that could heal quicker and better than other chronic wounds that could not heal.

For example, present techniques, such as wound clinical observation, fail to predict chronic or non-healing wound development and are insufficient to categorize patients with the healing outcome of the wound. If patients at risk of developing a chronic or non-healing wound could be identified, suitable preventive measures or treatments could be used in a targeted program of potentially great effectiveness.

WO 2011/033249 discloses a method and kit for the classification and prognosis of wounds based on molecular markers or genes.

However, there remains a need in the art for a method for the early diagnosis or prognosis of wound fate. In particular, there is a need for a sensitive and reliable method of diagnosing or prognosing of chronic or non-healing wounds such as, but not limited to, venous leg ulcers, diabetic foot ulcers, decubitus ulcers, and arterial leg ulcers, non-healing acute wounds.

It is therefore an object of the present invention to provide a method of diagnosis or prognosis of the outcome of the wound.

In a first aspect of the invention, a method of diagnosis or prognosis of a non-healing or chronic wound tissue is provided, said method comprising the step of determining the levels of expression of genes encoding different molecular markers in the wound from a mammalian, wherein said genes are defined as follows:
- at least one of the following genes show increased expression when compared with the expression in normal dermal fibroblasts of said mammalian:
   POU2F2, AMIGO2, CCL11, CDC45L, CSF2, CSF3, FOXS1, GOS2, IF44L, INHBA, KPRP, LCP1, LPAR3, MICAL2, MT1F, MT1M, POLQ, RRM2, SERPINA9, SOX9, STC1, TFIP2 and UCN2,
- or at least the following miRNA show decreased expression when compared with the expression in normal dermal fibroblasts of said mammalian:
   AC084368.1,
- or at least one of the following genes shows a normal expression when compared with the expression in normal dermal fibroblasts of said mammalian:
   CNN1 and KRT16,
- or at least one of the following genes show decreased expression when compared with the expression in normal dermal fibroblasts of said mammalian:
   ACTC1, ADAMTS7, CFB, COMP, ECM2, EDIL3, EFHD1, FOLR1, ITGA11, KIT, LBH, LGR5, MED12L, MFAP5, NR4A3, OMD, PALM, PHACTR3, PI16, PPARG, PTH1R, PTX3, RCAN2, RSPO1, SPON2, TAGLN, TMEM37, TMSB4Y, TXNIP and WFDC1,
- or at least one of the following miRNA show increased expression when compared with the expression in normal dermal fibroblasts of said mammalian:
   MIR147B and MIR1181.

In a preferred embodiment of the invention, the method of diagnosis or prognosis of a non-healing or chronic wound tissue also comprises the step of determining the levels of expression of genes encoding different molecular markers in a sample of a wound from a mammalian, wherein said genes are defined as follows:
- at least one of the following genes show decreased expression when compared with the expression in normal dermal fibroblasts of said mammalian:
   ACTC1, ADAMTS7, COMP, ECM2, EDIL3, EFHD1, FOLR1, ITGA11, LBH, LGR5, MED12L, MFAP5, OMD, PALM, PHACTR3, PI16, PTH1R, RSPO1, SPON2, TAGLN, TMEM37, TMSB4Y, TXNIP and WFDC1,
- or at least the following miRNA show decreased expression when compared with the expression in normal dermal fibroblasts of said mammalian:
   AC084368.1,
- or at least one of the following genes shows a normal expression when compared with the expression in normal dermal fibroblasts of said mammalian:
   CNN1 and KRT16,
- or at least one of the following genes show increased expression when compared with the expression in normal dermal fibroblasts of said mammalian:
   CCL11, CDC45L, CSF2, CSF3, GOS2, IF44L, KPRP, LCP1, LPAR3, MT1F, MT1M, POLQ, RRM2, SERPINA9, STC1 and TFIP2,
- or at least one of the following miRNA show increased expression when compared with the expression in normal dermal fibroblasts of said mammalian:
   MIR147B and MIR1181.

In a specific embodiment of the invention, the method of diagnosis or prognosis of a non-healing or chronic wound tissue also comprises the step of determining the levels of expression of genes encoding different molecular markers in a sample of a wound from a mammalian, wherein said genes are defined as follows:
- at least one of the following genes show decreased expression when compared with the expression in normal dermal fibroblasts of said mammalian:
   ACTA2, APOD, FGF9, ID4, POSTN and SMAD3,
- or at least one of the following genes show increased expression when compared with the expression in normal dermal fibroblasts of said mammalian:
   CXCL1, CXCL5, CXCL6, MMP10, MMP3, SERPINB2, SPHK1, HALPN1 and CTGF.

In another specific embodiment of the invention, the method of diagnosis or prognosis of a non-healing or chronic wound tissue also comprises the step of determining the levels of expression of genes encoding different molecular markers in a sample of a wound from a mammalian, wherein said genes are defined as follows:
- at least one of the following genes show decreased expression when compared with the expression in normal dermal fibroblasts of said mammalian:
   ACTA2, FGF9, ID4 and POSTN,
- or at least one of the following genes show increased expression when compared with the expression in normal dermal fibroblasts of said mammalian:
   CXCL1, CXCL5, CXCL6, MMP10, MMP3 and SERPINB2.

By "chronic wound" or "chronic wound tissue" or "non-healing wound", it is meant, for example, a disorder chosen from venous leg ulcers, diabetic foot ulcers, decubitus ulcers and arterial leg ulcers or non-healing acute wounds or non-healing wounds. The full identity of the genes according to the invention is available on the NCBI database (http://www.ncbi.nlm.nih.gov/), or is well known to those skilled in the art.

In a preferred aspect of the invention, the wound tissue is a human tissue, and the normal dermal fibroblasts are Normal Human Dermal Fibroblasts (NHDF).

In a preferred aspect of the invention, the normal dermal fibroblasts arise from the healthy skin of the said mammalian, and preferably the normal dermal fibroblasts arise from the healthy skin of the same animal or individual.

The term "determining the levels of expression of genes" as used above means qualitative and/or quantitative detection (measuring levels) with reference to a control. Typically the determination of the levels of expression of genes may be measured for example by RT-PCR performed on the sample or in situ hybridization or high-throughput sequencing, such as Lynx Therapeutics' Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope® single molecule sequencing, Single Molecule real time (RNAP), Single Molecule SMRT® sequencing, Nanopore DNA sequencing, VisiGen Biotechnologies approach.

Typically, said determination comprises contacting the sample with selective reagents such as probes, primers or ligands, and thereby detecting the presence, or measuring the amount of nucleic acids of interest originally present in the sample. Contacting may be performed in any suitable device, such as a plate, microtiter dish, test tube, well, glass or column. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column or a gel. The contacting may be made under any condition suitable for a detectable complex, such as a nucleic acid hybrid, to be formed between the reagent and the nucleic acids of the sample.

In a particular embodiment, the determination of the levels of expression of genes may be determined by quantifying the RNA of said genes. Said RNA are preferably chosen from mRNA and miRNA. Preferably, said RNA are mRNA.

Methods for measuring the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA may be then detected by hybridization (e. g., Northern blot analysis).

Alternatively, the extracted mRNA may be subjected to couple reverse transcription and amplification, such as reverse transcription and amplification by polymerase chain reaction (RT-PCR), using specific oligonucleotide primers that enable amplification of a region in the target gene. Preferably quantitative or semi-quantitative RT-PCR is used. Real-time quantitative or semi-quantitative RT-PCR is particularly advantageous. Extracted mRNA may be reverse-transcripted and amplified, after which amplified sequences may be detected by hybridization with a suitable probe or by direct sequencing, or high-throughput sequencing or any other appropriate method known in the art.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the RNA of interest herein find utility as hybridization probes or amplification primers. It is understood that such nucleic acids need not be identical, but are typically at least about 80% identical to the homologous region of comparable size, more preferably at least 85% identical and even more preferably at least 90%, preferably at least 95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 3x, 5x or 6x SCC. SCC is a 0.15 M NaCl, 0.015 M Na-citrate).

In the method of the invention, the presence of RNA, preferably total RNA, and more preferably the amount of mRNA, is assayed in the examined samples of wound tissue. All the techniques available for measuring RNA content can be used. Said techniques may include Northern blot, reverse transcription quantitative polymerase chain reaction, NanoString Technologies, microarray technology, or Serial Analysis of Gene expression (SAGE). In the present invention, high-throughput sequencing, such as Lynx Therapeutics' Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope® single molecule sequencing, Single Molecule real time (RNAP), Single Molecule SMRT® sequencing, Nanopore DNA sequencing, VisiGen Biotechnologies approach can also be used.

In an alternative embodiment of the invention, the determination of the levels of expression of genes in the sample may also be performed by quantifying the corresponding encoded proteins. All the techniques available for measuring protein content can be used.

Such methods comprise contacting a sample with a binding partner capable of selectively interacting with the target protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of antibodies against the proteins to be tested. A sample containing or suspected of containing the marker protein is then added to the coated wells. After a period of incubation sufficient to allow the formation of antibody-antigen complexes, the plate(s) can be washed to remove unbound moieties and a detectably labelled secondary binding molecule is added. The secondary binding molecule is allowed to react with any captured sample marker protein, the plate is washed and the presence of the secondary binding molecule is detected using methods well known in the art.

In an another aspect of the invention, there is provided a kit for performing any one or more of the aforementioned methods, wherein said kit comprises probes to detect and quantify the expression level of at least one target gene.

By "probes", it is meant single-stranded nucleic acids of between 10 to 1000 nucleotides in length, for instance of between 10 and 800, more preferably of between 15 and 700, typically of between 20 and 500, which hybridize with the target gene under high stringency hybridization conditions (corresponding to the highest melting temperature Tm, e.g., 50 % formamide, 3x, 5x or 6x SCC. SCC is a 0.15 M NaCl, 0.015 M Na-citrate).

According to a further aspect of the invention, there is provided a kit for performing any one of the aforementioned methods wherein said kit comprises:
(1)A plurality of probes for detecting and quantifying the expression level of all the genes specified in table 1,
(2) Optionally, reagents and instructions pertaining to the use of said probes.

In yet a further preferred aspect of the invention there is provided a kit for determining the prognosis of mammalian wound tissue which comprises:
(1)A plurality of probes for detecting and quantifying the expression level of at least one RNA or protein of each one of the genes of table 1,
(2) Optionally, reagents and instructions pertaining to the use of said probes.

Ideally, the instructions describe how to determine the expression level of each of said genes.

According to a further aspect of the invention there is provided a microarray comprising or consisting of any one or more of the aforementioned sets of probes. The kit according to the invention may use an apparatus such as the Ion Proton Sequencer of Life Technologies.

In another aspect of the invention, there is provided a kit for determining wound type in a patient, said kit comprising at least two microarrays, each comprising a plurality of probes for detecting and quantifying the expression level of all the genes specified in one of the above methods.

In a further aspect of the invention, there is provided a method for treating a wound which comprises the step of performing any one or more of the aforementioned methods for determining the classification or prognosis of wound tissue in order to identify whether said wound tissue is chronic or non-healing and selecting an appropriate treatment based on the classification or prognosis of the wound tissue.

In another aspect of the invention, there is provided a therapy consisting in increasing the expression of PI16 in chronic or non-healing wound. Said therapy may consist in the use of an activator of PI16 for treating chronic or non-healing wound.

### Role of fibroblasts in wound healing

Fibroblasts are implicated in the process of wound healing, this involves several steps of differentiation from a quiescent fibroblast to a mobilized fibroblast that will transform into a myofibroblast and finally enter apoptosis. In chronic or non-healing wounds, this process is misregulated and fibroblasts fail to undertake the myofibroblast differentiation and are found in the wound as unfunctional fibroblasts, called pseudo senescent fibroblasts (Telgenhoff D, Shroot B (2005) Cellular senescence mechanisms in chronic wound healing. Cell Death Differ 12: 695-698). The aim of the present invention is to map, at the whole genome scale, the different genes that will be activated or deactivated during this process, and thus providing a molecular signature of chronic or non-healing wounds.

Human fibroblasts have the ability to enter into a physiological process named senescence, which permits a limited replicative cell cycle and thus avoids loss of genetic information. It usually occurs when a cell has already conducted several rounds of replication (called replicative senescence and dependent from telomere length), but can also occur in response to environmental stress (Muller M (2009) Cellular senescence: molecular mechanisms, in vivo significance, and redox considerations. Antioxid Redox Signal 11: 59-98). Senescence cells are arrested in cell cycle but maintain metabolic activity (Telgenhoff D, Shroot B (2005) Cellular senescence mechanisms in chronic wound healing. Cell Death Differ 12: 695-698).

Fibroblasts of chronic wounds lose some of their functionalities, and more particularly, they lose part or all of their replicative function (Telgenhoff D, Shroot B (2005) Cellular senescence mechanisms in chronic wound healing. Cell Death Differ 12: 695-698). In a wound, human fibroblasts are also associated with an up-regulation of APA-1, a protein which induces matrix remodeling, demonstrating that pseudo-senescence fibroblast phenotype was not induced by telomere attrition (Benanti JA, Williams DK, Robinson KL, Ozer HL, Galloway DA (2002) Induction of extracellular matrix-remodeling genes by the senescence-associated protein APA-1. Mol Cell Biol 22: 7385-7397). Thus, senescent fibroblasts in chronic wounds would appear more particularly due to a chronic inflammation than to a telomere shortening (Telgenhoff D, Shroot B (2005) Cellular senescence mechanisms in chronic wound healing. Cell Death Differ 12: 695-698).

Some biological markers, such as TAGLN, are described in the prior art (Thweatt R, Lumpkin CK, Jr., Goldstein S (1992) A novel gene encoding a smooth muscle protein is overexpressed in senescent human fibroblasts. Biochem Biophys Res Commun 187: 1-7). In this publication, gene expression is increased in senescent cells whereas in the chronic or non-healing wound model used by the inventors, the gene expression of TAGLN is decreased when compared with normal fibroblast gene expression.

In (Yoon IK, Kim HK, Kim YK, Song IH, Kim W, Kim S, Baek SH, Kim JH, Kim JR (2004) Exploration of replicative senescence-associated genes in human dermal fibroblasts by cDNA microarray technology. Exp Gerontol 39: 1369-1378), GOS2 is described as being overexpressed in prepuce fibroblast senescent cells. However, in this article, the fibroblasts are in replicative senescence, obtained after more than twenty doubling population whereas in the present invention, as the inventors work on a chronic or non-healing wound model, the fibroblasts are pseudo-senescent and not in replicative senescence

Dermal fibroblast is a good experimental material since human cells can be obtained from different donors. By the way fibroblasts represent the key cells in wound healing, as they secrete the ECM proteins and differentiate in myofibroblasts that lead to the wound contraction.

Some biological markers are already described on fibroblasts from different tissues: for example, CCL11 in lung (Puxeddu I, Bader R, Piliponsky AM, Reich R, Levi-Schaffer F, Berkman N (2006), The CC chemokine eotaxin/CCL11 has a selective profibrogenic effect on human lung fibroblasts, J Allergy Clin Immunol 117: 103-110) or TFIP2 in synovial fibroblasts (Scaife S, Brown R, Kellie S, Filer A, Martin S, Thomas AM, Bradfield PF, Amft N, Salmon M, Buckley CD (2004) Detection of differentially expressed genes in synovial fibroblasts by restriction fragment differential display. Rheumatology (Oxford) 43: 1346-1352).

The legends of the figures are the following:
Figure 1: Schematic representation of the experiments performed with Human Normal Dermal Fibroblasts
Figure 2: levels of aSMA mRNA determined by quantitative RT-PCR in the different experiments
Figure 3: aSMA and tubulin expression determined by Western-Blot in the different experiments
Figure 4: Definition of the Lists II, III
Figure 5A : PI16 mRNA expression (mock siRNA or PI16 siRNA)
Figure 5B : aSMA mRNA expression (mock siRNA or PI16 siRNA)
Figure 6A : PI16 mRNA expression at different time point in T+E- condition
Figure 6B : PI16 mRNA expression at different time point in T-E+ condition
Figure 6C : PI16 mRNA expression at different time point in T+E+ condition
   Table 1: Gene signature list for the non-healing or chronic wounds
   Table 2: List of all genes transcripts identified in all the experiments performed (lists II and III).

### Example

In response to a lesion, fibroblasts migrate into the wound where they differentiate into contractile myofibroblasts that will finally enter into apoptosis during the remodeling phase. This differentiation process can be studied ex-vivo in environmentally controlled tissue culture conditions, and therefore the timely controlled succession of different gene expression patterns can be addressed.

### Materials and methods

### Establishment of an ex vivo model of chronic wounds

An ex vivo model of chronic or non-healing wounds was established by adding exudates from chronic wounds on cultured fibroblasts in order to reproduce the pathological state. Then, the gene expression was studied to define a molecular signature of chronic or non-healing wounds.

NHDF, isolated from human explants, were purchased from Promocell. NHDF were cultivated in DMEM-F12 (Invitrogen), supplemented with 10% FCS (Invitrogen, 5µg/mL of insulin and 1ng/mL of b-FGF (PromoKine)).

To collect exudates, four patients with mixed ulcers were recruited (mean age, 76 years; range 57-88 years). For patient selection, it was decided to exclude any other comorbidity factor potentially involved in wound etiology: diabetes, peripheral arterial diseases, malnutrition. Exudates were collected from negative pressure therapy. All the exudates were centrifuged at 1,500 x g for 3 minutes to remove cell debris. The supernatant was filtered and store at -80°C until use. Aliquots were used to determine protein concentration according to BCA method (Sigma).

For experiments, cells were deprived of insulin and b-FGF during 48 hours. Then, the cells were cultivated on collagen coated culture plates in DMEM-F12, supplemented with 10% FCS, 10ng/mL of TGF-β1 (Promocell) for 4 days. Four points were tested in order to appreciate the effect of exudate on fibroblast differentiation: untreated cells (T-E-), fibroblasts treated with TGF-β1 (T+E-), cells treated with exudate (T-E+) and finally fibroblasts treated with TGF-β1 and exudate at the same time (T+E+).

The efficiency of fibroblast differentiation was estimated by analyzing the expression of the myofibroblast marker alpha smooth muscle actin (aSMA).

This aSMA expression was assessed by RT-qPCR (mRNA levels) and by Western Blot (protein).

### Western Blotting assay

Total proteins were extracted by scratching the cells with lysis buffer (TRIS, NaCl, NP40, EDTA, IMDTT) and incubated 30 min in ice. To remove cell debris, the samples were centrifuged at 13,000 x g for 10 min at 4°C and store at -20°C until use. Protein concentration was determined according to BCA method (Sigma). Equal amounts of total protein (20µg) were loaded to NuPAGE 10% BIS-Tris gel (Invitrogen), separated by migration at 150 V, and transferred to nitrocellulose membrane (Whatman) 1 hour at 30 V. Then, membranes were stained for α-SMA (Abcam) and tubulin (Abcam). Incubations were followed by secondary antibodies goat anti-rabbit IgG and goat anti-mouse IgG, respectively, conjugated with horseradish-peroxidase (HRP) (Promega). Signals were detected by ECL chemiluminescence using UptiLight HS WB Substrate (Uptima, Interchim). Bands were digitized with a scanner and the ratio between all bands density of the same blot was calculated by software (ImageJ 1.43u, 64-bit). Relative α-SMA expression was normalized to the respective value for tubulin.

### Total RNA Sample Preparation

After four days of experiment, treated fibroblasts were lysed with TRIzol Reagent (Invitrogen) and stored at -80°C. Then RNA was purified using chloroform and precipitated by isopropanol. Total RNA was quantified on the NanoDrop 2000c Spectrophotometer (Thermo Scientific). Reverse transcription of 500 ng total RNA to cDNA was done with oligot dT (Invitrogen) using SuperScript III RT (Invitrogen) and RNAse OUT (Invitrogen). The cDNA was store at -20°C.

### Quantitative real-time RT-PCR

Quantitative real-time PCR (RT-qPCR) was done using 5µL of 1:20 diluted cDNA on the LightCycler480 system (Roche) using Maxima SYBR Green qPCR Master Mix (Fermentas). Forward and reverse primers were designed by Eurofins (MWG, aSMA forward: CTGTTTTCCCATCCATTGTG, aSMA reverse: CCATGTTCTATCGGGTACTT) and a 100µM stock was stored at -20°C. Forward and reverse primer pairs were used for each RT-qPCR reaction. The cycling conditions were as follows : an initial 95°C for 10 minutes, followed by 45 cycles of 95°C for 15 sec, 58°C for 30 sec, 72°C for 20 sec. LightCycler 480 SW 1.5 was used to evaluate the TM curves, to determine the Cp and to approximate the relative concentration for each amplification reaction.

### Timing of expression

NHDF (Normal Human Dermal Fibroblast) were treated with TGFbeta and/or exudate as described previously for different times (between 1h and 96h). After treatment mRNA were extracted and levels of PI16 mRNA were assessed by RTqPCR.

### siRNA Transfection

The expression of PI16 was knocked down by transiently transfecting human dermal fibroblasts with specific small interfering RNAs (Qiagen). Two different siRNAs were tested. For transfections, fibroblasts were trypsinized and seeded on collagen coated 6-well plates. TGF-β1 and/or exudates were added to the medium as described before. Then, NHDF were treated with 10nM siRNA and 4µL of INTERFERin reagent (PolyPlus), according to the manufacturer's instruction for 6 days. To maintain a sufficient knocking down, a second transfection was performed at 48h. The knockdown of target mRNA was confirmed by RT-qPCR. As a control, mock siRNA (directed against exogenous and non-present GFP mRNA) was used to bypass a possible effect of siRNA transfection into the cells.

### Results

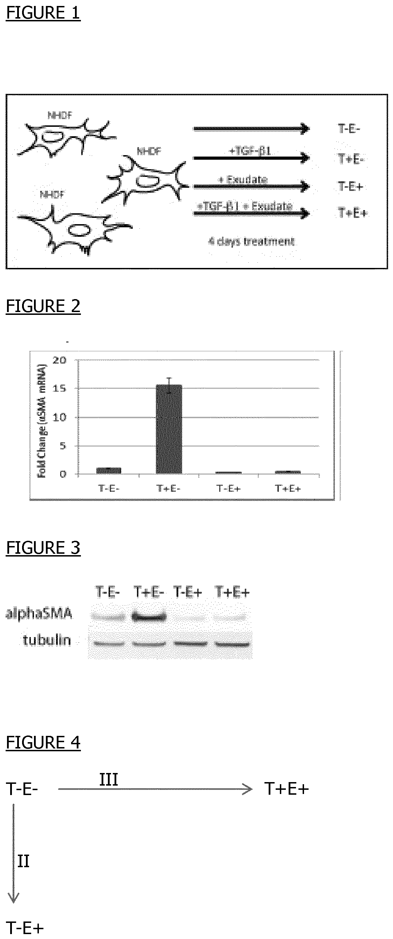

For the chronic or non-healing wound model, chronic wound exudates were added to cell cultures (500µg/mL of total proteins of exudate). The experiments that were performed are depicted in Figure 1: cells were either not treated (T-E-), either treated with TGFβ alone (T+E-), exudate alone (T-E+) or TGFβ and exudate (T+E+) for 4 days. The assays described previously were used to assess the level of differentiation. Chronic wound exudates decrease the expression of aSMA (mRNA and protein, figures 2 and 3). This indicated that chronic wound exudates clearly inhibit fibroblast differentiation. This is correlated to the fact that in chronic wounds, we can find unfunctional fibroblasts, also called pseudo-senescent fibroblasts.

In order to analyze the genes expressed upon different treatments of the fibroblasts in a chronic or non-healing wound model, mRNA deep sequencing was realized.

Total RNA was extracted by TRIzol. Equal amounts of total RNA of the different treated cells (5 to 6 µg) were precipitated by absolute ethanol, supplemented by sodium acetate for RNA sequencing.

The mRNA sequencing was performed by Fasteris SA (Switzerland). RNA was sent as total RNA, after two rounds of polyA purification, the Reverse transcription and the cDNA libraries were done. The sequencing was performed on a HiSeq2000 (Illumina).

One gene can contain different isoforms, and some isoforms can have one or more exons in common. Unfortunately, when the number of reads present in each isoforms is counted and fused in gene entities, sometimes the same reads may be counted several times and thus biases the analyses for genes with numerous isoforms. To solve this problem, it was decided to create a fictive transcript for each gene corresponding to the maximal portion of exon coverage, and to count the number of reads present in these entities. After the normalization and analysis of differential expression steps, only genes showing a differential expression associated with an adjusted p-value of 1.10⁻³ or less were retained. A supplementary filter on the logFC (Fold Change) to study complete lists (the absolute value of logFC has to be superior or equal to 2) was applied.

Pathologic wound healing analysis: chronic or non-healing wounds

The aim of the invention was to know if genes are differentially expressed between two conditions, in order to determine if the wound is a chronic or non-healing wound or not.

The abundance of gene transcripts between two conditions was compared, the T-E-point (normal dermal fibroblasts) being the reference. 2 lists (as defined in Figure 4) were considered: List II compared T-E+ and T-E- and List III compared T+E+ and T-E-. The comparison of the lists II and III with normal dermal fibroblasts provides the list of genes affected by chronic exudate during the process of differentiation, in fact genes affected in the pathological situation found in chronic wound healing. Thus, lists II and III represent the chronic or non-healing wound situation. With the p-value adjusted and the Log FC filters determined, 409 genes were identified as differentially expressed in list II and 1006 genes in list III.

Some genes, thanks to their high increased or decreased expression, are of particular interest. For example, the expression of PI16 is largely decreased in non-healing or chronic wounds.

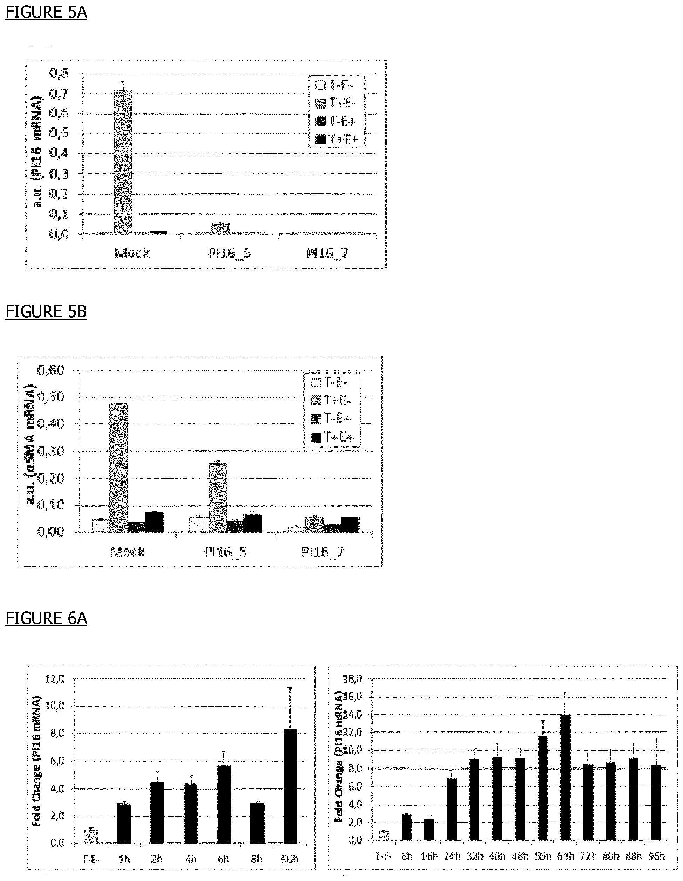

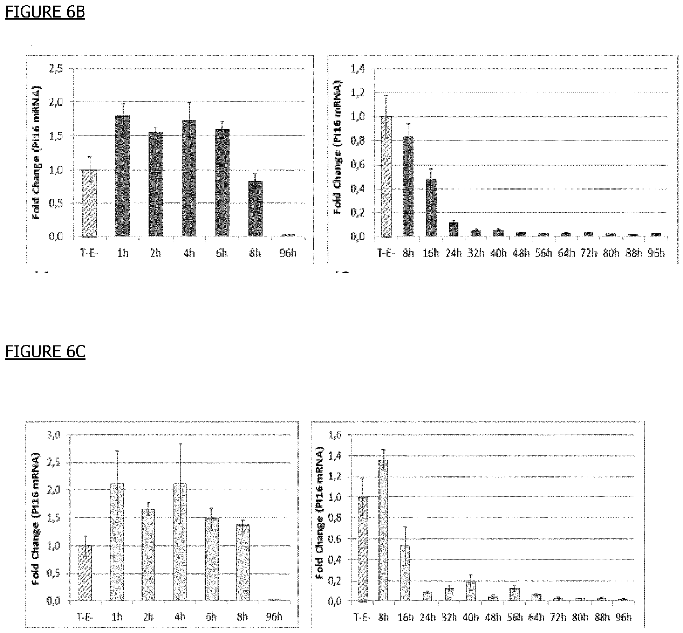

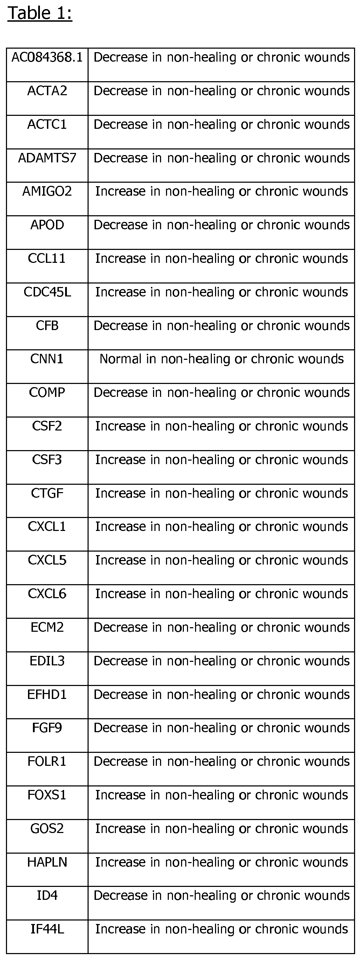

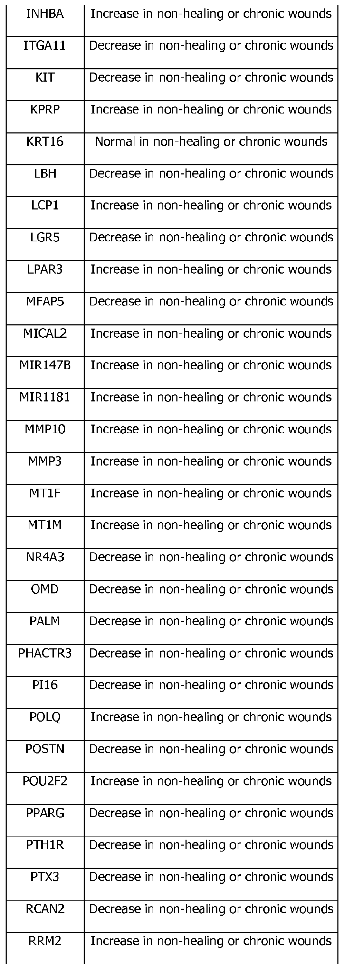

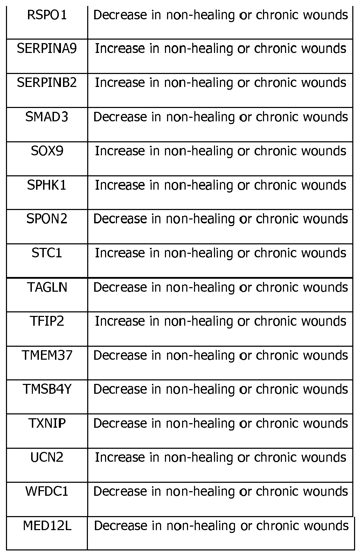

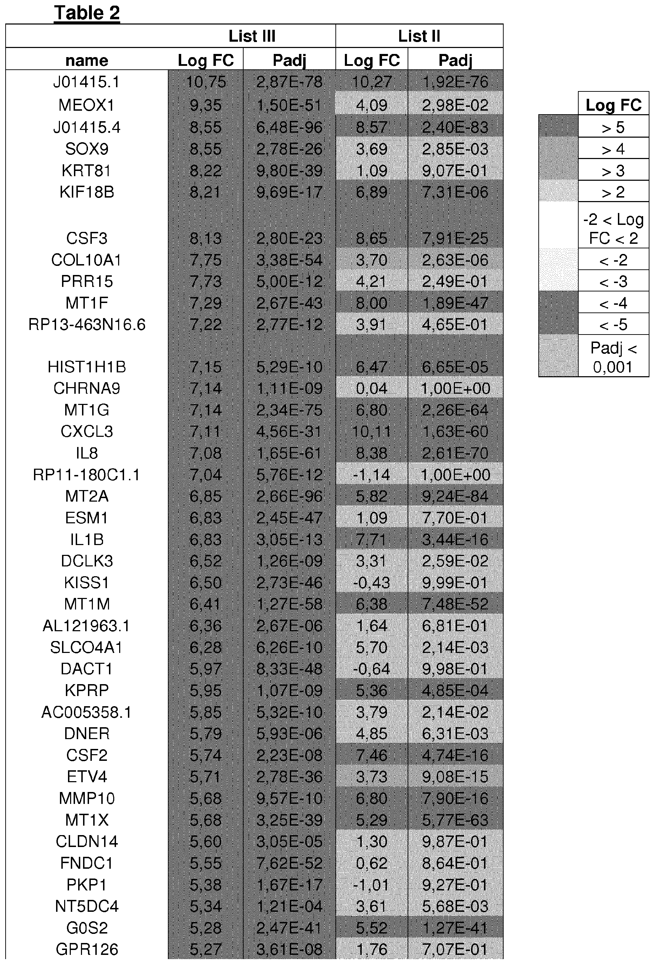

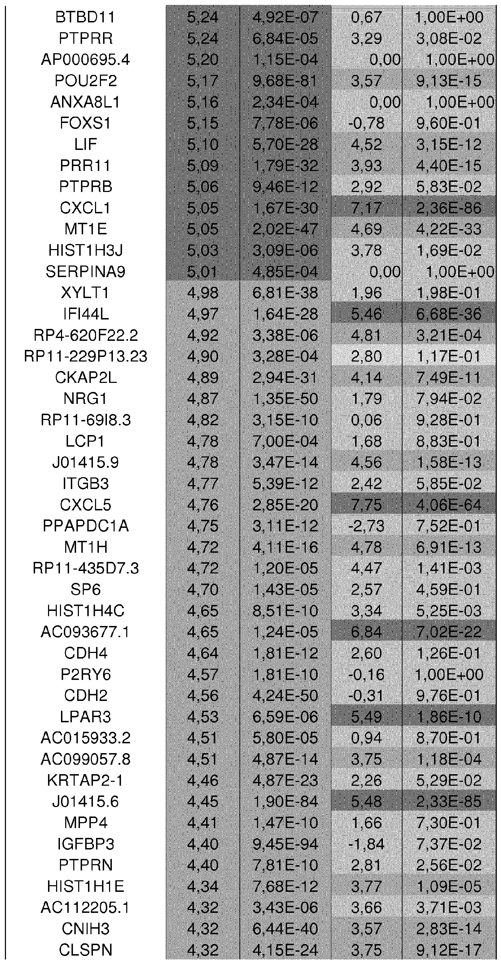

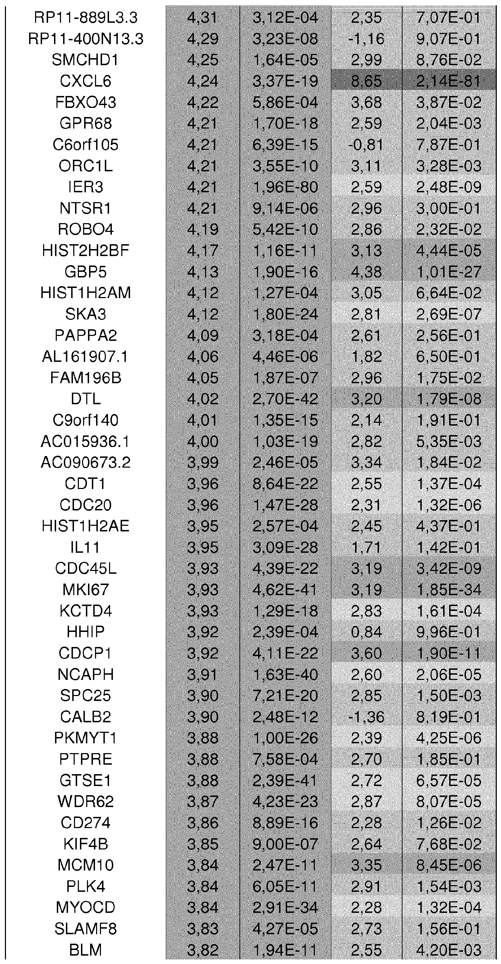

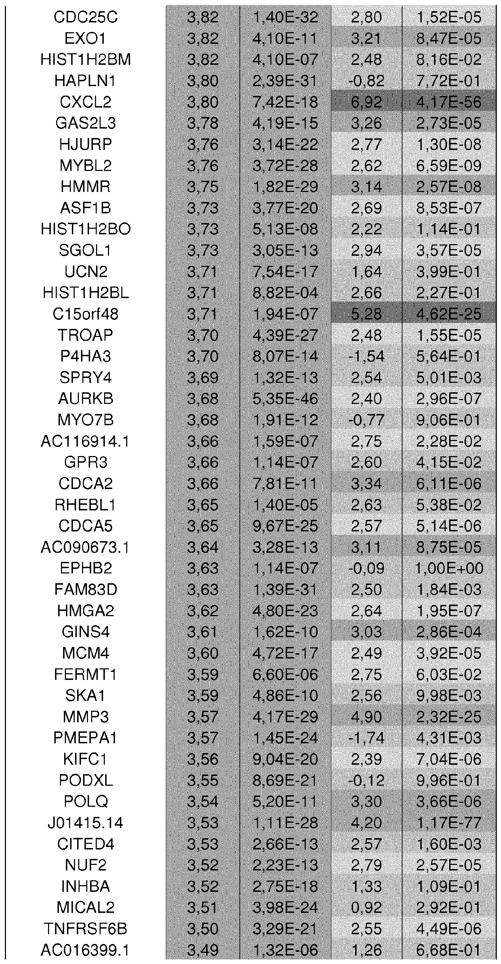

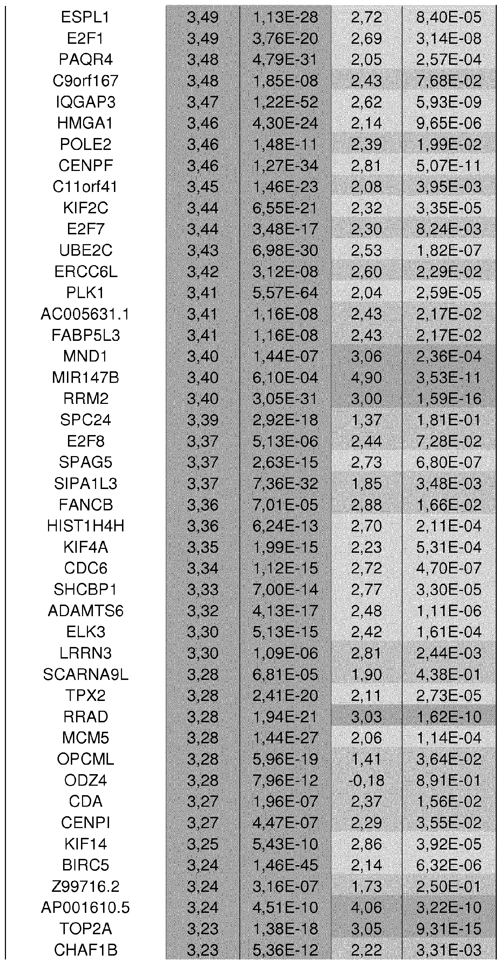

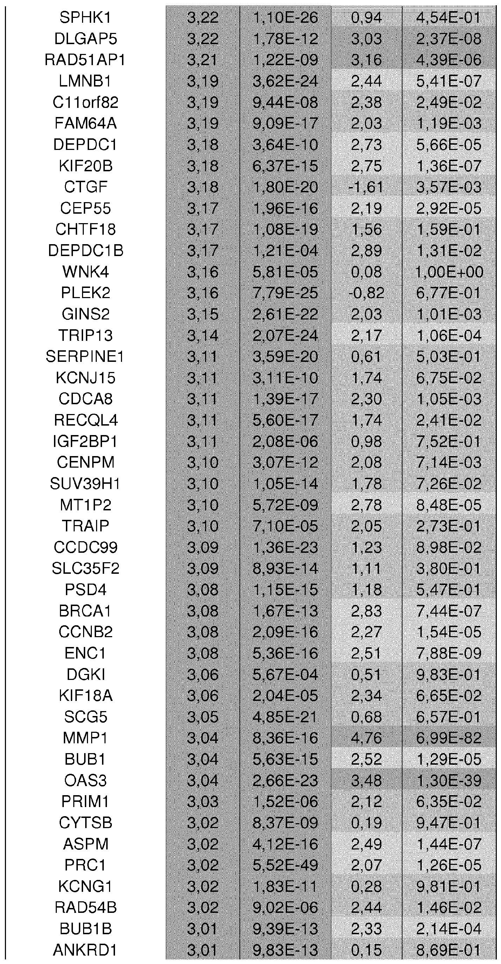

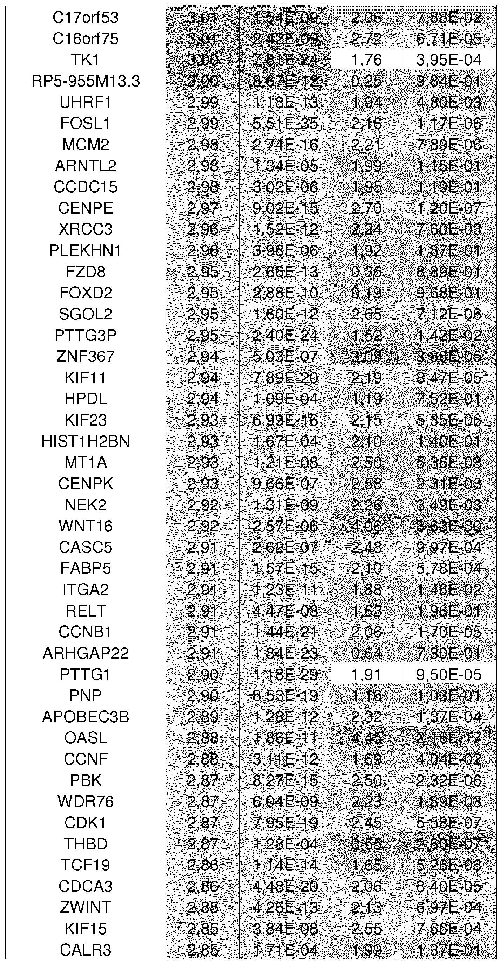

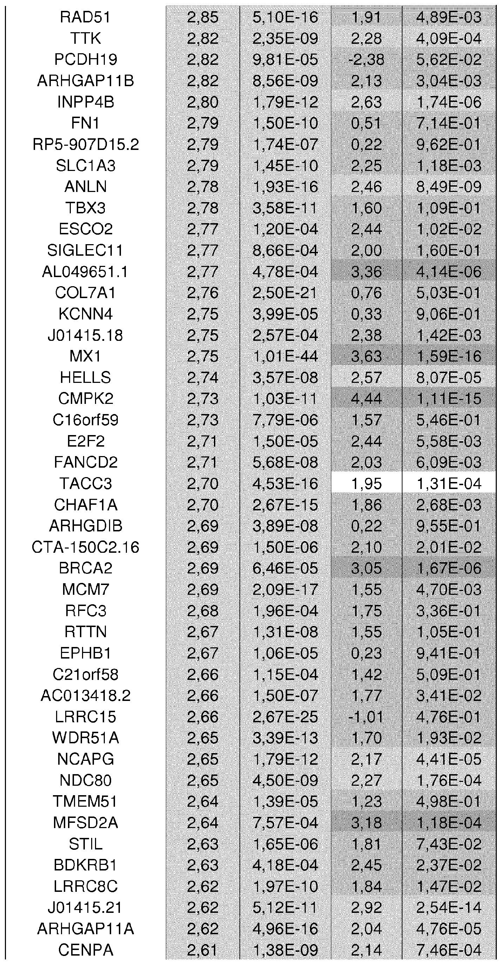

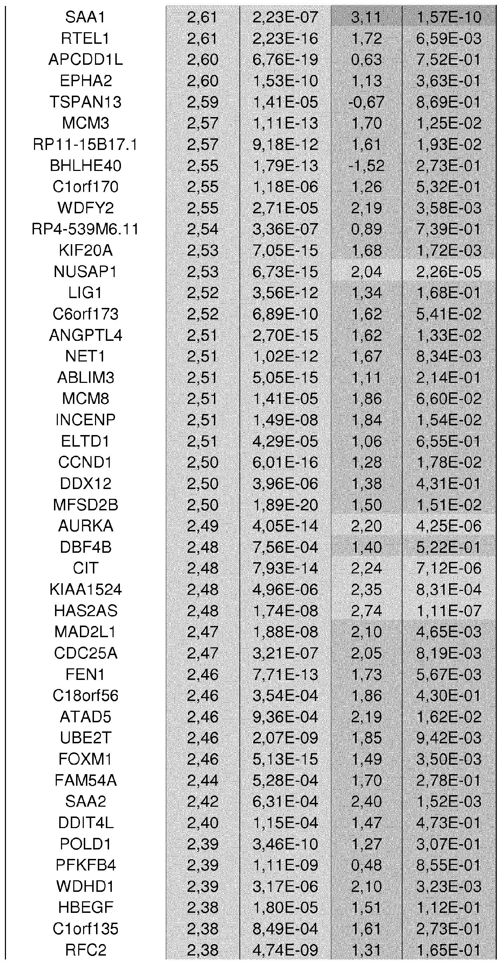

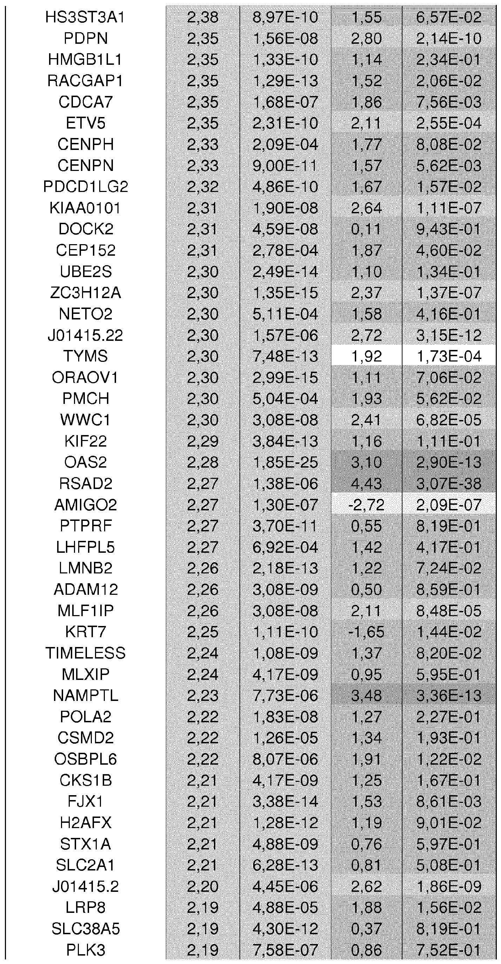

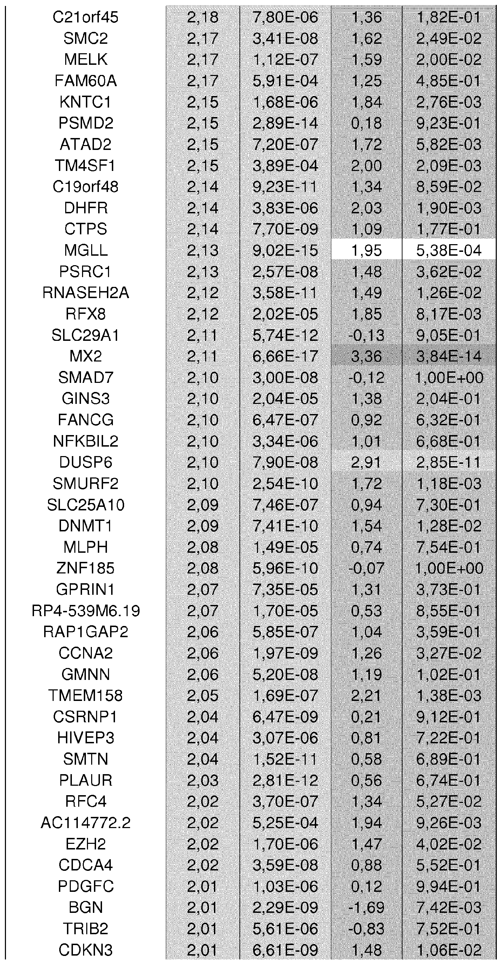

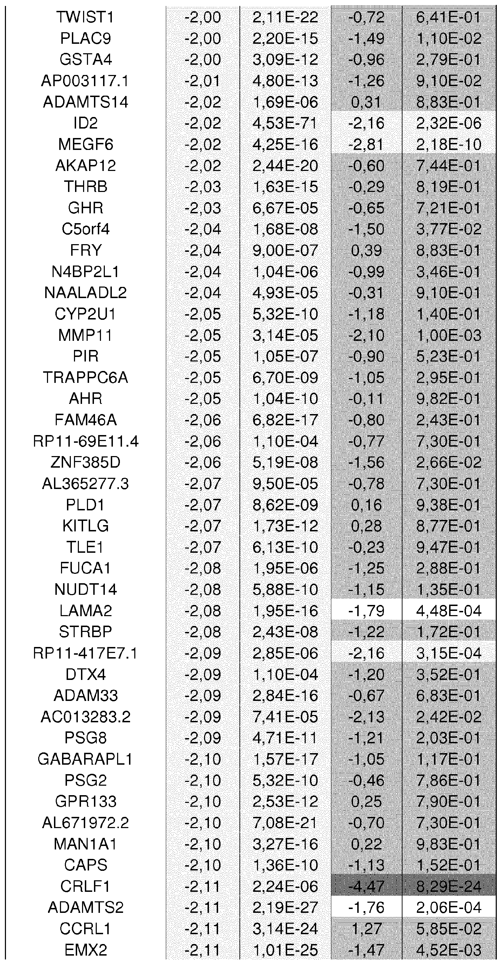

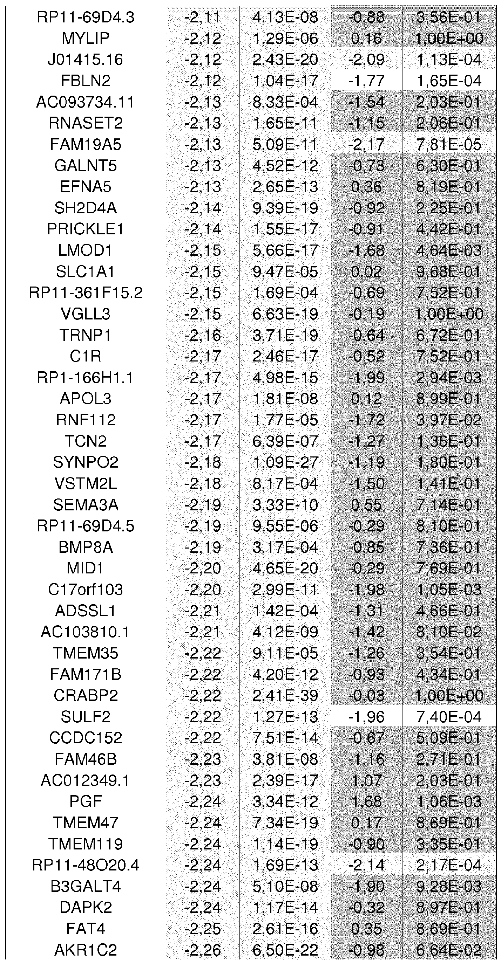

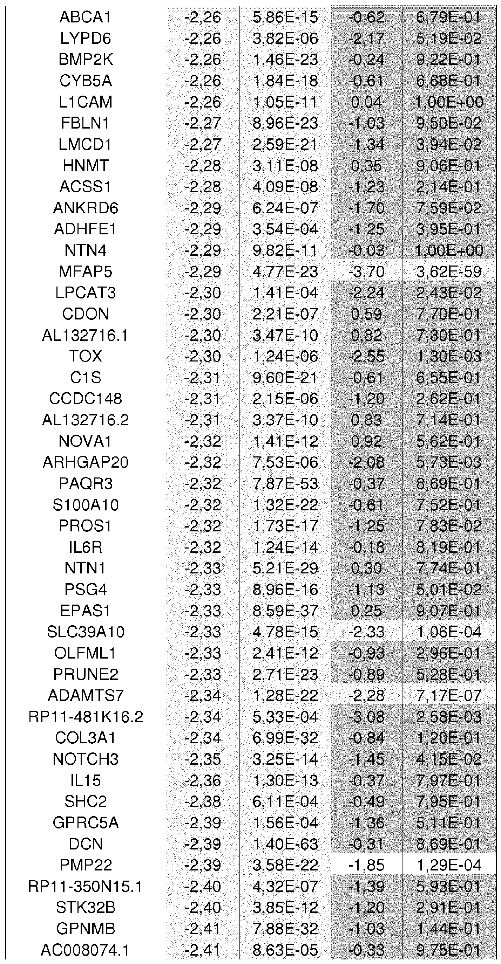

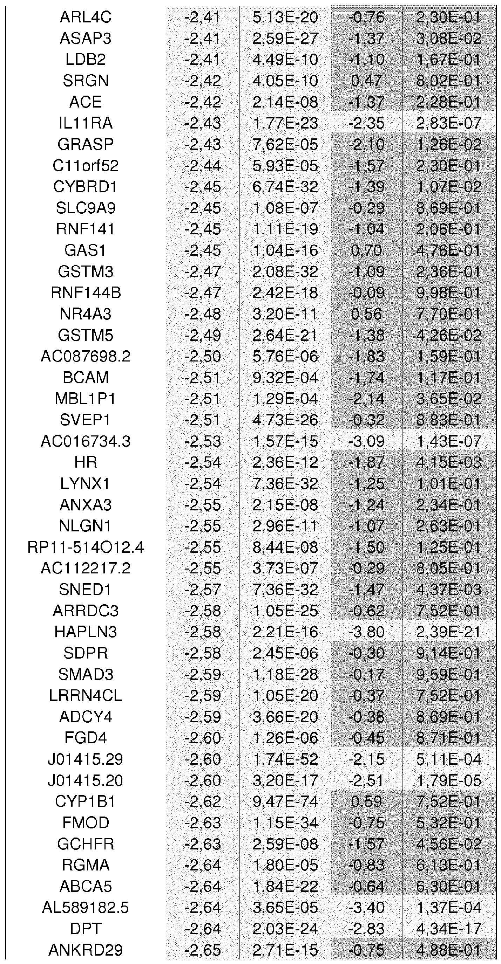

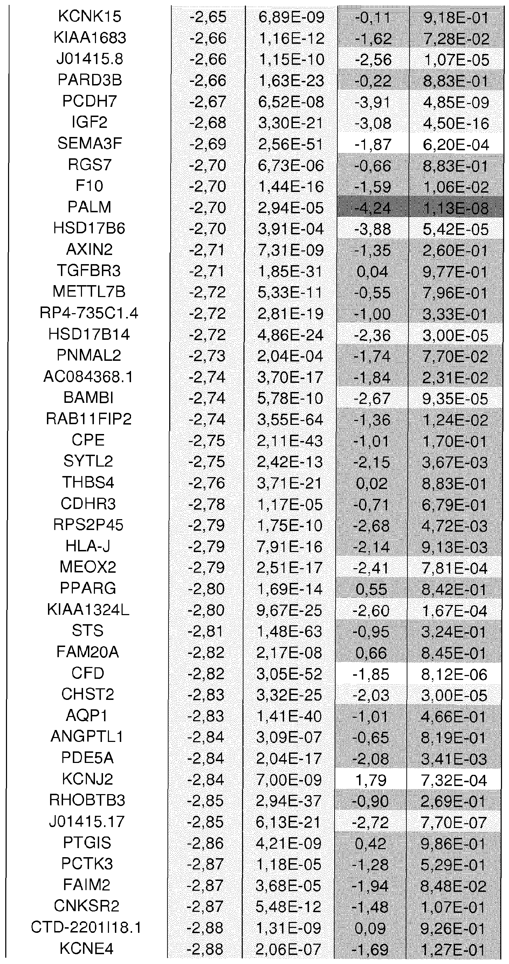

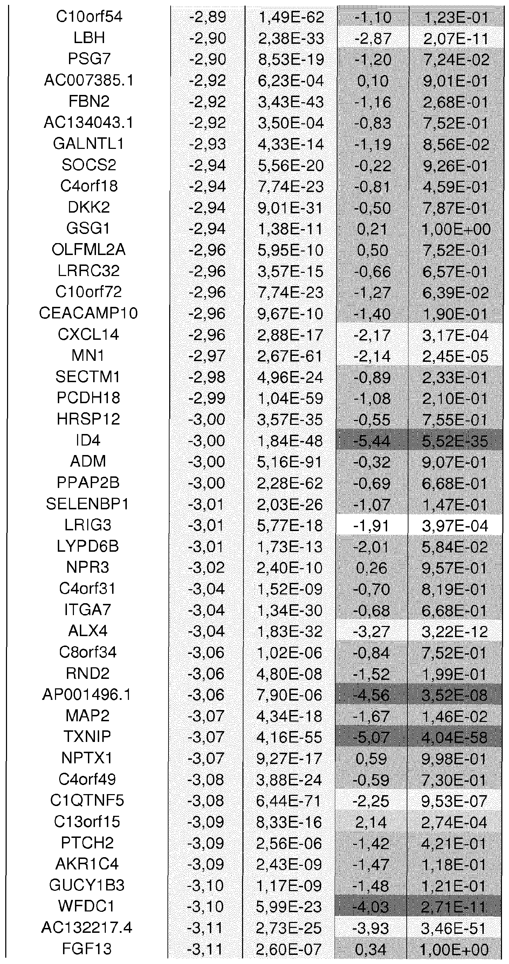

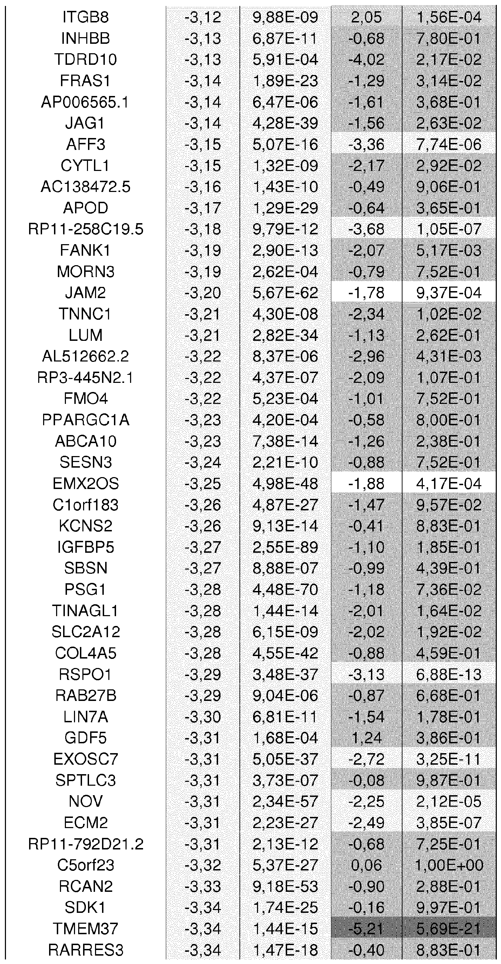

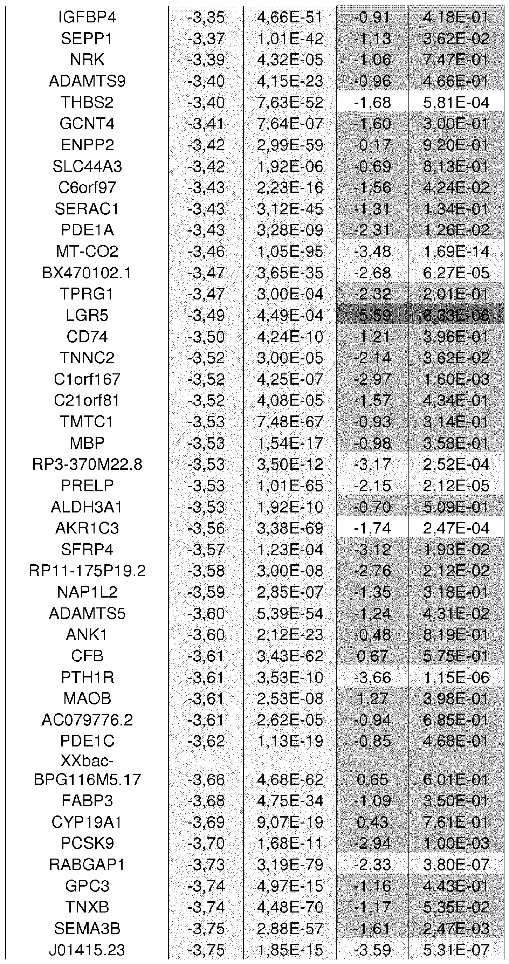

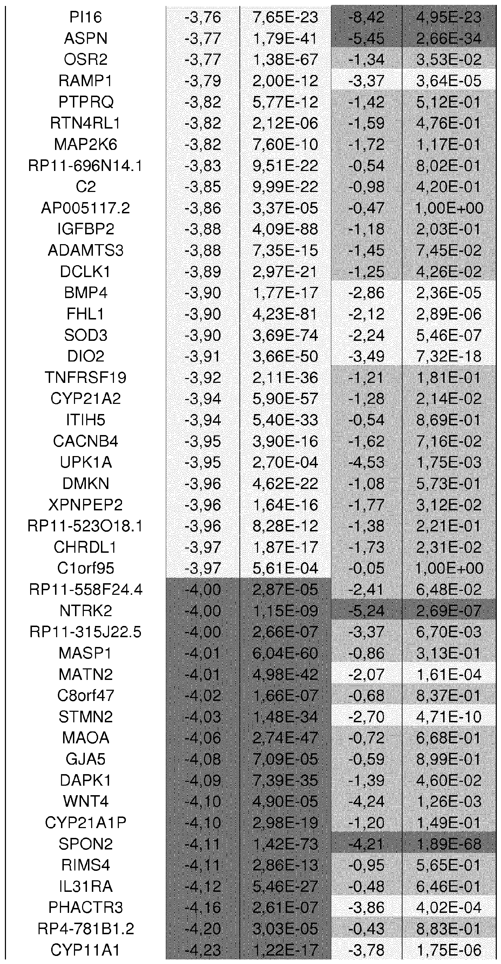

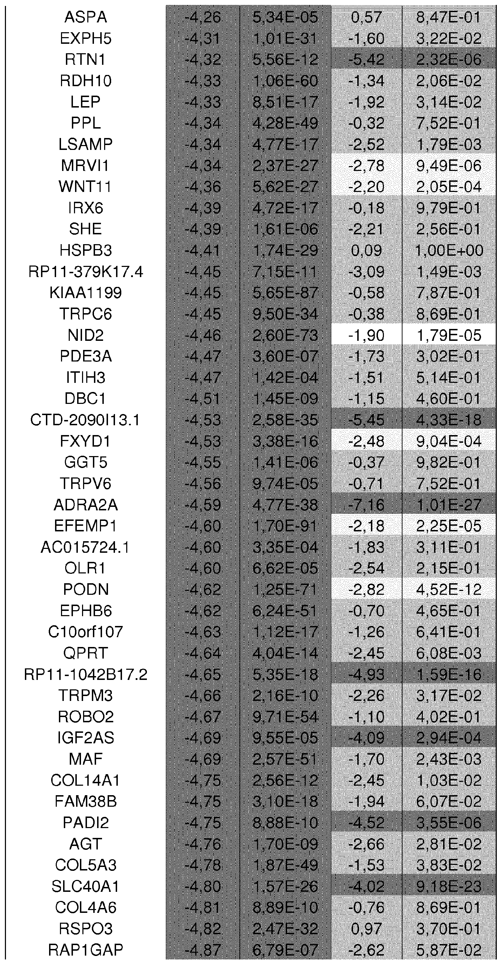

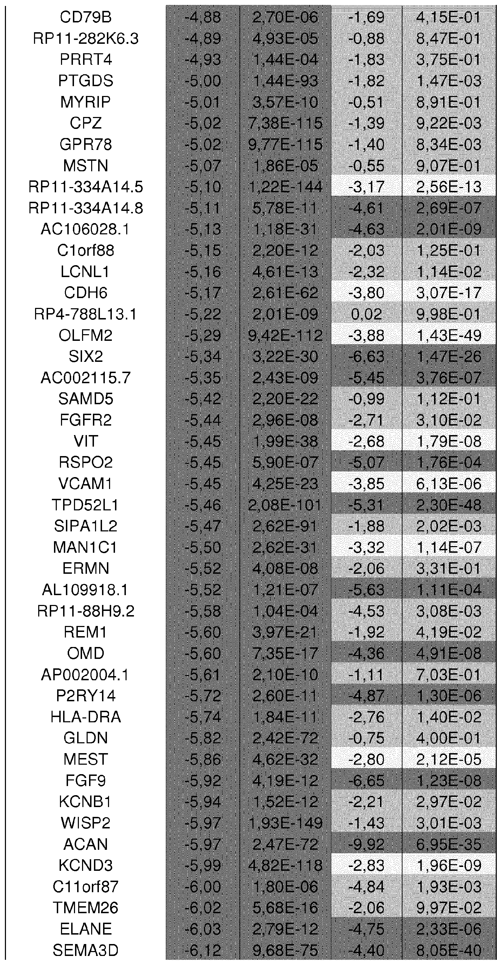

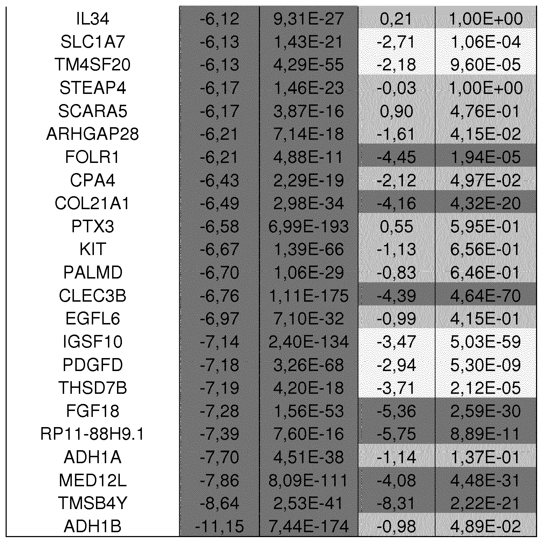

Whereas PI16 mRNA is usually overexpressed after TGFβ treatment, one can notice here the efficient knockdown of PI16 mRNA levels after siRNA treatment (Figure 5A). Very interestingly, when PI16 is downregulated, we can notice a total (for the siRNA PI16_7) or partial (for the siRNA PI16_5) inhibition of the TGFβ induced-levels of aSMA (Figure 5B) mRNA. These differences in the effect of the two siRNA can be correlated with the efficiency of the PI16mRNA knockdown. These results are in complete agreement with the expression pattern study of PI16 mRNA after TGFβ treatment. Indeed PI16 mRNA is largely up regulated after TGFβ treatment and completely down regulated (in a slower extend) with exudate treatment (Figures 6).

As a consequence we suggest that the overexpression of PI16 is associated with an increase in fibroblast to myofibroblast differentiation. On the contrary, a down regulation of PI16 correlates with a non-differentiation behavior of the fibroblast (with exudate treatment or siRNA approaches).Thus, PI16 is a favorite candidate for therapy. The present invention also directed to a therapy consisting in increasing their expression in chronic or non-healing wound condition.

## Claims

1. A method of diagnosis or prognosis of a non-healing or chronic wound tissue comprising the step of determining the levels of expression of genes encoding different molecular markers in a sample of a wound from a mammalian, wherein:
at least PI16 gene shows decreased expression when compared with the expression in normal dermal fibroblasts of said mammalian.

2. A method of diagnosis or prognosis of a non-healing or chronic wound tissue according to claim 1, further comprising the step of determining the levels of expression of genes encoding different molecular markers in a sample of a wound from a mammalian, wherein:
- at least one of the following genes show decreased expression when compared with the expression in normal dermal fibroblasts of said mammalian:
ACTC1, ADAMTS7, COMP, ECM2, EDIL3, EFHD1, FOLR1, ITGA11, LBH, LGR5, MED12L, MFAP5, OMD, PALM, PHACTR3, PTH1R, RSPO1, SPON2, TAGLN, TMEM37, TMSB4Y, TXNIP and WFDC1,
- or at least the following miRNA show decreased expression when compared with the expression in normal dermal fibroblasts of said mammalian:
AC084368.1,
- or at least one of the following genes shows a normal expression when compared to the expression in normal dermal fibroblasts of said mammalian:
CNN1 and KRT16,
- or at least one of the following genes show increased expression when compared with the expression in normal dermal fibroblasts of said mammalian:
CCL11, CDC45L, CSF2, CSF3, GOS2, IF44L, KPRP, LCP1, LPAR3, MT1F, MT1M, POLQ, RRM2, SERPINA9, STC1 and TFIP2,
- or at least one of the following miRNA show increased expression when compared with the expression in normal dermal fibroblasts of said mammalian:
MIR147B and MIR1181.

3. A method according to claim 1 or 2 wherein, furthermore:
- at least one of the following genes shows decreased expression when compared with the expression in normal dermal fibroblasts of said mammalian:
ACTA2, APOD, FGF9, ID4, POSTN and SMAD3,
- or at least one of the following genes show increased expression when compared with the expression in normal dermal fibroblasts of said mammalian:
CXCL1, CXCL5, CXCL6, MMP10, MMP3, SERPINB2, SPHK1, HALPN1 and CTGF.

4. A method according to one of the proceeding claims wherein, furthermore:
- at least one of the following genes show decreased expression when compared with the expression in normal dermal fibroblasts of said mammalian:
ACTA2, FGF9, ID4 and POSTN,
- or at least one of the following genes show increased expression when compared with the expression in normal dermal fibroblasts of said mammalian:
CXCL1, CXCL5, CXCL6, MMP10, MMP3 and SERPINB2.

5. A method according to any proceeding claims, wherein said wound tissue is human wound tissue, and normal dermal fibroblasts are Normal Human Dermal Fibroblasts (NHDF).

6. A method according to any proceeding claims, wherein the normal dermal fibroblasts arise from the healthy skin of the said mammalian and more preferably the wound tissue and the normal dermal fibroblasts arise from the same animal or individual.

7. A method according to any proceeding claims, wherein the said levels of expression of genes are determined by quantifying the corresponding RNA.

8. A method according to claim 7, wherein said RNA is chosen from mRNA and miRNA.

9. A method according to any one of claims 1-8, wherein the said levels of expression of genes are determined by quantifying the corresponding encoded proteins, except for the miRNA expression.

10. A method according to claim 9, wherein said proteins are measured by using antibodies.

11. A kit for performing any one or more of the aforementioned methods according to any one of claims 1-10, wherein said kit comprises:
(1) A plurality of probes for detecting and quantifying the expression levels of all the genes specified in table 1,
(2) Optionally, reagents and instructions pertaining to the use of said probes.

12. A kit for determining the prognosis of mammalian wound which comprises:
(1) A plurality of probes for detecting and quantifying the expression level of at least one RNA or protein of each one of the genes of table 1,
(2) Optionally, reagents and instructions pertaining to the use of said probes.

13. A microarray consisting of any one or more of the sets of probes in claims 11-12.

14. A kit for determining a wound outcome in a patient, comprising:
at least two microarrays comprising a plurality of probes for detecting and quantifying the expression levels of all the genes specified in any one of claims 1 to 10.
